# EUROPEAN PATENT SPECIFICATION

(11) **EP 2 701 711 B1**
(45) Date of publication and mention of the grant of the patent: **30.09.2020**
(21) Application number: 12776559.2
(22) Date of filing: 24.04.2012
(51) Int. Cl.: A61K 31/7012, A61K 31/7016, A61K 31/70, A61P 25/00, A61K 31/7008, A23L 33/125, A23L 33/105, A23L 29/30, A23L 33/10, A23L 5/00, A61P 25/28

(54) **ACUTE COGNITIVE AND MOOD EFFECTS OF PLANT POLYSACCHARIDES IN ADULT HUMAN SUBJECTS**
AKUTE KOGNITIVE UND STIMMUNGSAUFHELLENDE EFFEKTE PFLANZLICHER POLYSACCHARIDE BEI ERWACHSENEN PERSONEN
EFFETS COGNITIFS ET SUR L'HUMEUR AIGUS DE POLYSACCHARIDES DE PLANTE CHEZ DES SUJETS HUMAINS ADULTES

(30) Priority: 27.04.2011 US 201161479632 P; 23.04.2012 US 201213453540
(43) Date of publication of application: 05.03.2014
(73) Proprietor: Mannatech, Inc., Coppell, TX 75019 (US); University of South Australia, Adelaide, South Australia 5001 (AU)
(72) Inventor: BEST, Talitha, Happy Valley South Australia 5159 (AU)
(74) Representative: Sonn & Partner Patentanwälte
(86) International application number: PCT/US2012/034752
(87) International publication number: WO 2012/148887

(56) References cited:
- WO-A1-98/06418
- BEST TALITHA ET AL: "Saccharide Effects on Cognition and Well-Being in Middle-Aged Adults: A Randomized Controlled Trial", DEVELOPMENTAL NEUROPSYCHOLOGY, vol. 35, no. 1, 17 December 2009 (2009-12-17), pages 66-80, XP9180309, ISSN: 8756-5641, DOI: 10.1080/87565640903325709
- STANCIL ATIYA N ET AL: "Glyconutrients and perception, cognition, and memory.", PERCEPTUAL AND MOTOR SKILLS FEB 2009, vol. 108, no. 1, February 2009 (2009-02), pages 259-270, XP009180330, ISSN: 0031-5125
- BLOOMER RICHARD J ET AL: "Effect of Ambrotose AOÂ TM on resting and exercise-induced antioxidant capacity and oxidative stress in healthy adults", NUTRITION JOURNAL, BIOMED CENTRAL, GB, vol. 9, no. 1, November 2010 (2010-11), page 49, XP021077651, ISSN: 1475-2891, DOI: 10.1186/1475-2891-9-49
- Anonymous: "Ambrotose Complex powder", MannatechScience, 23 September 2014 (2014-09-23), pages 1-2, XP055142173, Retrieved from the Internet: URL:http://mannatechscience.org/home/produ cts/ambrotose [retrieved on 2014-09-23]
- RODRÍGUEZ RODRÍGUEZ ELENA ET AL: "Aloe vera as a functional ingredient in foods", CRITICAL REVIEWS IN FOOD SCIENCE AND NUTRITION, TAYLOR & FRANCIS, USA, vol. 50, no. 4, April 2010 (2010-04), pages 305-326, XP009180332, ISSN: 1040-8398, DOI: 10.1080/10408390802544454

## Description

### Technical Field of the Invention

The present invention relates in general to the field of dietary supplements, and more particularly, to a dietary supplement comprising plant polysaccharides that has a beneficial effect on cognitive performance and mood in middle-aged adults.

### Background Art

Without limiting the scope of the invention, its background is described in connection with health effects of plant polysaccharides including dietary supplements thereof.

U.S. Patent No. 7,157,431 issued to Mcanalley et al. (2007) discloses compositions of plant carbohydrates for dietary supplements and nutritional support for promotion and maintenance of good health. Defined nutritionally effective amounts of one to eleven essential saccharides, glyconutrients, are used in various inventive compositions as dietary supplements. The dietary composition herein can include phytonutrients, vitamins, minerals, herbal extracts, and other non-toxic nutrients. The glyconutritional dietary supplement herein provides essential saccharides which are the building blocks of glycoproteins. These compositions, when administered orally or topically, have been found to improve the well being of mammals suffering from a variety of disorders.

Best et al (Dev Neurophychol, 2009, 35(1):66-80) discloses the long-term effects of the administration of Ambrotose® Complex on cognition and well-being in middle-aged adults.

### Disclosure of the Invention

The present invention describes the beneficial health effects of dietary supplements containing plant polysaccharides. More specifically, the present disclosure details improved cognitive performance and mood in middle-aged adults following administration of a plant polysaccharide containing dietary supplement.

The instant invention provides a dietary supplement for use in a method of improving recognition memory performance in a human subject by selecting a human subject in need of improvement of recognition memory performance; and administering to the human subject a nutritionally effective amount of the dietary supplement in an amount sufficient to improve recognition memory performance; waiting 30 minutes to allow the dietary supplement to be absorbed; and obtaining an improvement in recognition memory performance in the human subject compared to a recognition memory performance in a human subject who consumed sucrose or a placebo as a dietary supplement wherein the improvement in recognition memory performance occurs within 30 minutes to two hours after the dietary supplement has been administered; wherein the dietary supplement comprises a nutritionally effective amount of isolated and purified acetylated man-nose; and a nutritionally effective amount of at least five isolated and purified saccharides selected from the group consisting of: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan. Disclosed is a method for improving cognition, mood, learning, memory, reducing stress, anxiety, mental-fatigue, modifying behavior, or any combinations thereof in a human subject comprising the steps of: identifying the human subject in need of improvement of cognition, mood, learning, memory, reduction of stress, anxiety, mental-fatigue, modifying behavior, or any combinations thereof and administering a nutritionally effective amount of a dietary supplement in an amount sufficient to improve cognition, mood, learning, memory, reduce stress, anxiety, mental-fatigue, modifying behavior, or any combinations thereof. In one aspect of the instant invention, the dietary supplement is adapted for oral administration and is in the form of a powder, is dissolved in a liquid, is encapsulated, or any combinations thereof.

The method, as described hereinabove, further comprises the steps of: (i) performing one or more tests to assess the cognition, memory, mood, stress and anxiety level, or any combinations thereof in the human subject prior to the administration of the dietary supplement to determine a baseline or pre-test level for the human subject, (ii) performing one or more tests to assess the cognition, memory, mood, stress and anxiety level, or any combinations thereof in the human subject at one or more specified time-points after the administration of the dietary supplement to determine a post-test level for the human subject, and (iii) comparing the baseline and the post-test levels to determine continuation, termination, or modification of the administration of the dietary supplement in the human subject.

In another aspect, the human subject may suffer from one or more heart conditions, diabetes, head/brain injury, neurological conditions, neurodegenerative conditions, psychiatric conditions, or any combinations thereof. In yet another aspect, the stress or the anxiety arises from one or more stress disorders, Post Traumatic Stress Disorder (PTSD), phobias, psychological traumas, or any combinations thereof. In another aspect, the dietary supplement alleviates one or more adverse effects induced by central nervous system drugs, alcohol abuse, or any combinations thereof.

The method, described above, further comprises the step of measuring blood glucose levels prior to and after administration of the dietary supplement. In another aspect, the dietary supplement does not cause an elevated blood glucose level in the subject and the dietary supplement may be administered simultaneously or sequentially in one or a combination of dosage forms. In yet another aspect, the dietary supplement may be administered simultaneously or sequentially with one or more drugs, vitamins, other nutritional supplements, or any combinations thereof.

The dietary supplement described in the method hereinabove comprises: (i) a nutritionally effective amount of isolated and purified acetylated mannose and (ii) a nutritionally effective amount of at least five isolated and purified saccharides selected from: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan.

A method for improving cognition, mood, learning, memory, reducing stress, anxiety, mental-fatigue, modifying behavior, or any combinations thereof in a human subject without elevation of blood glucose levels is described. The method comprises the steps of: identifying the human subject in need of improvement of cognition, mood, learning, memory, reduction of stress, anxiety, mental-fatigue, modifying behavior, or any combinations thereof and administering a nutritionally effective amount of a dietary supplement in an amount sufficient to improve cognition, mood, learning, memory, reduce stress, anxiety, mental-fatigue, modifying behavior, or any combinations thereof. In one aspect, the dietary supplement is adapted for oral administration. In another aspect, the dietary supplement is a powder, is dissolved in a liquid, is encapsulated, or any combinations thereof. In yet another aspect, the method comprises the step of measuring blood glucose levels prior to and after administration of the dietary supplement and the step of terminating the administration of the dietary supplement in case of an abnormally elevated blood glucose levels.

The method, as described hereinabove, further comprises additional steps, these include: (i) performing one or more tests to assess the cognition, memory, mood, stress and anxiety level, or any combinations thereof in the human subject prior to the administration of the dietary supplement to determine a baseline or pre-test level for the human subject, (ii) performing one or more tests to assess the cognition, memory, mood, stress and anxiety level, or any combinations thereof in the human subject at one or more specified time-points after the administration of the dietary supplement to determine a post-test level for the human subject, and (iii) comparing the baseline and the post-test levels to determine continuation, termination, or modification of the administration of the dietary supplement in the human subject. In one aspect, the human subject may suffer from one or more heart conditions, diabetes, head/brain injury, neurological conditions, neurodegenerative conditions, psychiatric conditions, or any combinations thereof. In another aspect, the stress or the anxiety arises from one or more stress disorders, Post Traumatic Stress Disorder (PTSD), phobias, psychological traumas, or any combinations thereof. In yet another aspect, the dietary supplement alleviates one or more adverse effects induced by central nervous system drugs, alcohol abuse, or any combinations thereof.

The dietary supplement of the method of the present invention may be for administration simultaneously or sequentially in one or a combination of dosage forms and with one or more drugs, vitamins, other nutritional supplements, or any combinations thereof. The dietary supplement comprises: (i) a nutritionally effective amount of isolated and purified acetylated mannose and a nutritionally effective amount of at least five isolated and purified saccharides selected from: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan. Disclosed is that the at least five isolated and purified saccharides further comprise glucosamine and rhamnose.

Disclosed is a method for improving cognition, mood, learning, memory, reducing stress, anxiety, mental-fatigue, modifying behavior, or any combinations thereof in a human subject without elevation of blood glucose levels comprising the steps of: identifying the human subject in need of improvement of cognition, mood, learning, memory, reduction of stress, anxiety, mental-fatigue, modifying behavior, or any combinations thereof and administering a nutritionally effective amount of a dietary supplement in an amount sufficient to improve cognition, mood, learning, memory, reduce stress, anxiety, mental-fatigue, modifying behavior, or any combinations thereof. The dietary supplement disclosed herein comprises: a nutritionally effective amount of isolated and purified acetylated mannose and a nutritionally effective amount of at least five isolated and purified saccharides selected from: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan.

### Description of the Drawings

For a more complete understanding of the features and advantages of the present invention, reference is now made to the detailed description of the invention along with the accompanying figures and in which:
FIG. 1 is a schematic representation of the test protocol showing the running order for the testing day in hours;
FIG. 2 is a plot showing the participants subjective ratings of mental fatigue post treatment; and
FIG. 3 is a plot showing the effects of polysaccharides, placebo and sucrose on blood glucose levels.

### Description of the Invention

While the making and using of various embodiments of the present invention are discussed in detail below, it should be appreciated that the present invention is defined in the claims.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

As used herein, the term "carbohydrate" is used interchangeably with the terms "saccharide", "polysaccharide", "oligosaccharide" and "sugar" the definitions of which are well known in the art of carbohydrate chemistry. Although the compositions of the invention are intended to include at least one of the eleven essential saccharides, it should be noted that the saccharides can be in the form of mono-, oligo- and/or polysaccharides, e.g. a composition containing gum tragacanth and guar gum will be considered as containing galacturonic acid, fucose, xylose, arabinose, rhamnose, mannose and galactose. Therefore, by controlling the amount of particular gums in a given dietary supplement, one can control the amount of the respective saccharides in said dietary supplement.

Although the present invention includes the above cited eleven essential saccharides, it should be noted that other saccharides, nutritional compounds or biologically active or inert compounds can be included in the dietary supplement of the invention. Such other nutritional compounds include any one or more of phytonutrients, dioscorea complex, plant extracts, herbal extracts, plant parts, herbal components, vitamins or minerals. These nutritional compounds can be added to the dietary supplement of the invention, or they can be provided separately to a mammal being administered said dietary supplement.

There are many plant and herbal extracts with suspected or demonstrated nutritional value which can promote good health and can be incorporated in or administered along with the dietary supplement of the invention. Such plant and herbal extracts can be obtained according to well known procedures for the extraction of substances, compounds or agents from plants or herbs. Many different types of vitamins and minerals can be included in the dietary supplement of the invention. While a few vitamins and minerals of synthetic origin do possess nutritional value, particular embodiments of the dietary supplement herein contain nutritionally effective amounts of non-toxic vitamins and minerals obtained predominantly from natural sources.

The term "nutritionally effective amount" as used herein refers to that amount which will provide a beneficial nutritional effect or response in a mammal. For example, as nutritional response to vitamin- and mineral-containing dietary supplements varies from mammal to mammal, it should be understood that nutritionally effective amounts of said vitamins and minerals will vary, respectively. Thus, while one mammal may require a particular profile of vitamins and minerals present in defined amounts, another mammal may require the same particular profile of vitamins and minerals present in different defined amounts.

Other compounds, agents and nutrients can also be included in the dietary supplement of the invention, such as, for example, cellulose, calcium carbonate, kola nut, kola nut extract, country mallow, Atlantic kelp, cayenne pepper, silica, stearic acid, amino acids, glycine, lysine, glutamic acid, arginine, calcium carbonate, orchic substances, boron citrate, chromium picolinate, essential fibers, essential oils, essential botanicals, essential enteric ecology and flora growth promoters, essential fatty acids, live probiotic flora, proteins and enzymes.

The dietary supplement of the invention has been prepared and administered to mammals in powdered, reconstitutable powder, liquid-solid suspension, liquid, capsule, tablet, caplet, lotion and cream dosage forms. It should be readily obvious to one of ordinary skill in the science of formulations that the present dietary supplement can also be formulated appropriately for irrigation, ophthalmic, otic, rectal, sublingual, transdermal, buccal, vaginal, or dermal administration. Thus, other dosage forms such as chewable candy bar, concentrate, drops, elixir, emulsion, film, gel, granule, chewing gum, jelly, oil, paste, pastille, pellet, shampoo, rinse, soap, sponge, suppository, swab, syrup, chewable gelatin form, or chewable tablet can be used.

Due to varying diets among people, the dietary supplement of the invention can be administered in a wide range of dosages and formulated in a wide range of dosage unit strengths. For example, for those people who are missing from their diet nine of the eleven essential saccharides, a dietary supplement containing those nine saccharides in nutritionally effective amounts can be formulated. As well, for those people whose bioabsorption of essential saccharides is extremely efficient, a dietary supplement formulation containing reduced amounts of essential saccharides can be prepared.

It should be noted that the dosage of the dietary supplement can also vary according to a particular ailment or disorder that a mammal is suffering from when taking the supplement. For example, a person suffering from chronic fatigue syndrome, or fibromyalgia, will generally require a dose different than an alcoholic who is trying to discontinue alcohol consumption in order to obtain a nutritional benefit. An appropriate dose of the dietary supplement can be readily determined by monitoring patient response, i.e., general health, to particular doses of the supplement. As well, when another agent such as a phytonutrient, plant extract, herbal extract and/or dioscorea complex is being administered to a mammal along with the present glyconutritional dietary supplement, the appropriate doses of the supplement and each of the agents can be readily determined in a like fashion by monitoring patient response, i.e. general health, to particular doses of each.

It is contemplated by the invention that the dietary supplement can be administered simultaneously or sequentially in one or a combination of dosage forms. The present glyconutritional dietary supplement will provide a beneficial nutritional response in a mammal consuming it.

The present invention describes the beneficial effects of plant polysaccharides on cognitive performance and mood in middle-aged adults. The method of the present invention uses a randomized, double-blind, placebo-controlled design to study the acute effects of a unique combination of plant polysaccharides on tasks of memory, high cognitive demand serial subtraction tasks (Serial Threes and Serial Sevens), and a Rapid Visual Information Processing (RVIP) task in adults aged 45-60 years. The results from this study show beneficial effects of acute consumption of plant polysaccharide on memory performance and tasks of high cognitive demand. Importantly, these enhancement effects were independent of blood glucose responses. Overall, the findings of the present invention suggest that polysaccharides enhance memory.

The dietary supplement of the present invention comprises a nutritionally effective amount of isolated and purified acetylated mannose and a nutritionally effective amount of at least five isolated and purified saccharides selected from: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan. In one aspect of the present, invention the at least five isolated and purified saccharides are essential saccharides and further comprise glucosamine and rhamnose.

The acetylated mannose and the at least five isolated and purified saccharides are provided in monomeric, oligomeric and/or polymeric forms and may be available in the powdered form, dissolved in a liquid or is encapsulated.

It will be understood by the skilled artisan that the acetylated mannose and the saccharides may be obtained from a variety of natural sources. Non-limiting examples for the source of the acetylated mannose is the group consisting of aloe vera and acetylated polymannose, and the saccharides may be obtained from the group consisting of gum tragacanth, guar gum, grain flour, rice flour, sugar cane, beet sugar, potato, milk, agar, algin, locust bean gum, psyllium, karaya gum, seed gums, Larch tree extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, maltose, glucan, lentinan, mannan, levan, hemicellulose, inulin, fructan, and lactose.

In addition to improving cognitive performance and mood, the present inventors hypothesise that the dietary supplement may be used for the modification of behavior in alcohol dependent mammals comprising nutritionally effective amounts of the natural and/or synthetic monomeric, oligomeric and/or polymeric forms of acetylated mannose, gum ghatti, gum tragacanth, glucosamine, corn starch and arabinogalactan. The dietary supplement may reduce the craving for alcohol in an alcohol dependent mammal being administered the supplement. In another particular embodiment, the dietary supplement may improve the overall well being of the alcohol dependent mammal by reducing at least one of depression and anger or increasing at least one of cognition, energy and positive outlook.

The dietary supplement disclosed herein may reduce the undesired side-effects in mammals receiving biologically effective agents that cause said side-effects, said dietary supplement comprising nutritionally effective amounts of the natural and/or synthetic monomeric, oligomeric and/or polymeric forms of acetylated mannose, gum ghatti, gum tragacanth, glucosamine, corn starch and arabinogalactan. The dietary supplement may reduce the undesired side-effects of central nervous system drugs.

The studies conducted in the present invention employed a randomized, double-blind, placebo controlled, parallel groups design. Participants were randomly assigned to receive the plant polysaccharide mixture or, alternatively, a sucrose or starch placebo.

Volunteers for the study were recruited through word of mouth, television and print media from local metropolitan communities in Adelaide and Melbourne. The sample consisted of healthy middle-aged men and women aged between 45 and 60 yrs. Inclusion criteria included proficiency in English, no history of major heart surgery, diabetes, or taking medications for head/brain injury or neurological or psychiatric conditions that, could affect cognitive performance.

The study presented herein excluded volunteers having a history of one or more stress disorders, post traumatic stress disorder (PTSD), phobias, psychological traumas, alcohol abuse, and adverse reactions to one or more central nervous system (CNS) drugs. However, it will be understood that the dietary supplement described hereinabove is expected to have beneficial effects on cognition and mood of subjects suffering from the one or more exclusion conditions of this study.

There were 93 individuals enrolled in the study: 19 individuals withdrew prior to their first visit due to work commitments, inability to keep the appointment visit time, and concurrent life/family demands; and 1 individual who no longer wanted to complete the activities withdrew consent half-way through the assessment. There were 73 volunteers that completed the acute intervention study.

Prior to participation, volunteers provided written informed consent and completed the background health questionnaire to determine age, height, and weight in order to calculate body mass index (kg/m²), years of education, number of hours of work per week, self-rated health, (rated on a scale from 1= poor to 5 = excellent), number of dietary supplements used, number of medications used, number of hours of exercise per week, number of cigarettes smoked per day and alcohol consumption (number of standard drinks consumed in a typical day). Descriptive statistics of the three treatment groups are presented in Table 1. In addition, participants also completed the Profile of Mood state questionnaire (POMS) and Short-form health survey (SF-36) to determine background mood and well-being. The study was approved by the University of South Australia and Swinburne University of Technology Human Research Ethics Committees.

Procedure: Each participant attended the research facility on a single day for a period of 3.5 hours during which they completed 2 testing sessions, 1 baseline test and 1 post-treatment test.

All testing took place between 08:00 - 12:00 and 13:00-18:00. On testing days, participants abstained from consuming any stimulants (i.e., tea, coffee, other caffeine containing products) for 2 hours before their visit. All volunteers were fasted for 2 hours before their visit. Each study day comprised of a practice module, a pre-treatment baseline testing session on the mood and cognitive measures, this was immediately followed by a pre-treatment blood glucose measurement, and administration of treatment.

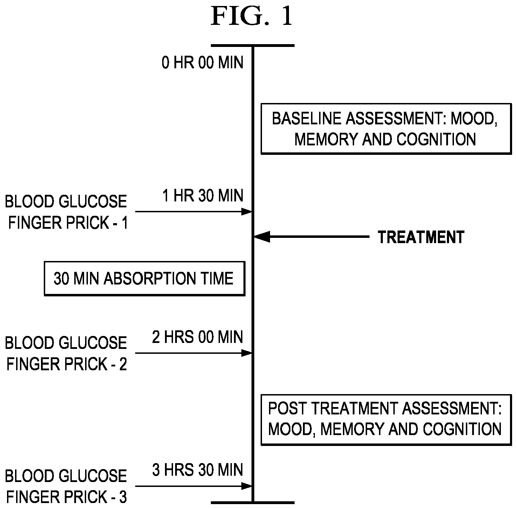

Participants were allocated, using a random number generator randomization code, to one of three treatment conditions: sucrose (icing sugar), placebo (rice flour) and plant saccharides (Ambrotose ®Complex). Given the different densities of the three supplement conditions, each treatment was weighed and measured with scoop amounts to provide equated 4 g doses. The supplement was delivered to each participant on a tablespoon with 100 ml of water. Each participant was instructed by the inventors to carefully consume the powder, without breathing in or out over it, by putting it in their mouth and washing it down with the water provided within 5 minutes. Additional water was provided on request. Participants were then instructed to rest for 30 minutes to allow for absorption. Following the 30 minute absorption time, a finger-capillary blood glucose measurement was taken. The post treatment testing session then commenced with alternate forms of the RAVLT and Cognitive demand battery. Following completion of the testing session, a final blood glucose measurement was taken. FIG. 1 shows the running order for the study day.

**Table 1: Baseline health and demographic means for each of the three treatment groups.**

| **Background Variable** | **Treatment Condition** | | |
|---|---|---|---|
| | **Polysaccharides N= 23** | **Sucrose N= 24** | **Placebo N= 26** |
| Gender | M = 8, F = 15 | M = 9, F = 17 | M = 9, F = 15 |
| Age in years | 53(4.41) | 52 (4.35) | 53 (4.49) |
| BMI (kg/m²) | 27 (4.48) | 29(8.00) | 26 (3.82) |
| Years of Education | 16 (4.70) | 15(3.32) | 15(3.99) |
| Self rated health | 4.0 (.90) | 4.9 (.62) | 4.1 (.56) |
| Hrs of work/week | 23.5 (15.81) | 21.4(19.04) | 19.5 (18.24) |
| Hrs of exercise/week | 4.5 (4.01) | 4.3 (3.77) | 4.4 (3.49) |
| No# supplements | 1.91(1.37) | 2.66 (2.28) | 2.86(1.88) |
| No# medications | 1.61(1.19) | 1.38 (.76) | 1.62(.51) |
| No# of Alcoholic drinks/week | 5.7 (6.18) | 5.5 (6.11) | 5.2(4.56) |
| No# of glasses of water /day | 3.7 (2.38) | 3.8 (2.12) | 4.1 (4.14) |

### Measures: Learning and Memory and Cognitive Measures

Learning and Memory: The Rey Auditory-Verbal Learning Test (RAVLT) [1] was used to assess immediate recall, delayed recall, learning and recognition memory. The examiner reads aloud 15 nouns (list A) over five trials and after each trial, participants are asked to recall, in any order, as many of the 15 words as possible. Scores for the 5 trials are summed to produce a measure of immediate recall, the difference between trial 5 and 1 is used as a measure of learning. After a sixth trial consisting of 15 different words (list B) participants are again required to recall the words that were presented in list A (trial 7), and then again after an interval of 60 minutes (trial 8). The scores from trials 7 and 8 are used to produce a measure of delayed recall, the difference between the words recalled in trials 8 and 7 is used as a measure of forgetting, the difference between trial 7 and trial 5 is used as a score of interference. After trial 8, participants are presented with a sheet of 50 words containing the words from lists A and B among 20 distracter words. Participants are asked to recognize the words from lists A and B and indicate the list they came from. Each word correctly identified is awarded one point producing a measure of recognition.

Cognitive Demand Battery: The computerized battery comprises of 6 repetitions of a 10 min assessment that measures the speed, accuracy and mental fatigue of performance during continuous, cognitively demanding tasks. Each 10 min cycle of this battery comprises two serial subtraction tasks (Serial Threes - 2 min, and Serial Sevens - 2 min), a Rapid Visual Information Processing Task (RVIP- 5 min) and Visual analogue mental fatigue scale (1 min). Tasks within this battery have been shown to be sensitive to the effects of other nutritional interventions, such a Panax ginseng and cocoa [2, 3].The individual tasks are described below.
a) *Serial Threes subtraction task* (2 min): Participants are required to count backwards in threes from a random starting number between 800 and 999 presented on the computer screen. Participants are instructed to enter their answer as quickly and as accurately as possible by using the linear number keys at the top of the computer keyboard. The starting number is cleared from the screen once participants enter their first response. Each subsequent three digit response by participants is presented on the screen as asterisks. Participants press the enter key to signal the end of each response and clear the three asterisks from the screen. The task is scored for the number of correct and incorrect responses, average reaction time, and proportion of correct responses made given average reaction time (accuracy vs speed).
b) *Serial Sevens subtraction task* (2 min): This task is identical to the serial threes task except that participants are asked to subtract sevens.
c) *Rapid visual Information Processing Task* (RVIP - 5 min): Participants are required to monitor a continuous series of digits for target strings of three consecutive odd or three consecutive even digits. The digits are presented on the screen at a rate of 100 per minute. The participant responds by pressing the space bar as quickly as possible when they detect a target string of digits. The task is scored for number of target strings correctly detected, percentage of correct answers, number of false alarms, average reaction time for correct detections and proportion of correct responses made given average reaction time (accuracy vs speed).
d) 'Mental-fatigue' visual analogue scale (1 min): Participants rate their subjective feelings of mental fatigue on a 100 mm visual analogue scale with anchors of 'not at all' and 'very much so'.

Mood and Well-being Measures: Background general mood and well-being were assessed with the Profile of Mood States (POMS) and Sf-36 health survey to determine any background differences between the groups. Subjective mood states on the testing day that may be effected by treatment conditions were assessed with 1 paper-pencil State-trait anxiety questionnaire, and 1 visual analogue scale that measures three mood states, alertness, calmness and contentedness.
(i) The POMS [4] is a self-report questionnaire that contains 65 items pertaining to six mood states: tension-anxiety, depression-dejection, anger-hostility, vigour-activity, fatigue-inertia, and confusion-bewilderment. Participants are asked to rate these on a 5-point scale (0= not at all to 4= extremely) indicating how they have felt during the past week including today.
(ii) The SF-36 health survey [5] provides a measure of functional physical and psychological health across seven general health and well-being concepts: 1) limitations in physical activities because of health problems; 2) limitations in social activities because of physical or emotional problems; 3) limitations in usual role activities because of physical health problems; 4) general mental health (psychological distress and well-being); 5) limitations in usual role activities because of emotional problems; 6) vitality (energy and fatigue); and 7) general health perceptions.
(iii) The State-trait anxiety questionnaire [6] contains 20 items that record the presence (e.g. 'I am tense') and absence (e.g. 'I feel at ease') of anxiety symptoms. Participants are asked to rate each item according to how they are generally feeling on a four-point scale ranging from 'not at all' or 'almost never' to 'very much so" or 'almost always'. These are combined to provide a sum score between 20 and 80 (a lower score representing lower anxiety).
(iv) The Bond-Lader visual analogue scale (VAS) [7] consists of 16 x 100 mm visual analogue scales. Participants indicate their current subjective state of mood for the 16 pairs of linked antonyms (e.g., attentive-dreamy, trouble-tranquil, happy-sad, alert-drowsy) by using the computer mouse to click on the line at a position that reflects their levels of mood. The resultant scores are combined to provide measures of self-rated alertness, calmness and contentedness.

Blood Glucose Measurement: Blood glucose samples were measured using an automated analyzer (Accu-check, optimal blood glucose monitor and testing strips). Blood samples were collected using self administered single use-capillary, disposable blood sampling lancet tips. Alcohol soaked cleansing swabs were used for pre-sampling sterilisation. The blood glucose reading were assessed and recorded at three testing points: pre-treatment, post-treatment and post cognitive testing.

Statistical Analysis: The primary outcomes were memory performance, mood and performance on the Cognitive Demand Battery (CDB) and the subjective ratings of mental fatigue. All data were analyzed using SPSS 17 statistical package as "change from pre dose baseline scores', using Analysis of Variance (ANOVA). To test for chance baseline differences which may have skewed change-from-baseline scores, prior to the primary statistical analysis, one-way ANOVA's with Bonferroni correction, were run all on all baseline demographics, general mood and well-being measures, and all pre-dose baseline measures to determine any potential covariates in outcomes.

Baseline health and wellbeing: There were no significant differences between groups on background demographic measures, Table 1. There was a significant difference between groups on the POMS scale of anxiety (*F* (3, 68) = 3.25, *p* = .044), seen in Table 2. Post hoc comparisons revealed that no group was significantly different from another, however the direction of means suggested that the placebo group was less anxious than those in the polysaccharide and sucrose groups (*p* = .06 and .08 respectively).

Therefore the anxiety sub-scale from the POMS was used as covariate in analysis for any differences between the groups on the baseline and post treatment measures of memory, cognitive demand tasks and subjective mood ratings.

There were no significant differences between groups on measures of memory or subjective mood ratings at baseline. There was a marginally insignificant difference between groups on the percentage correct and number correct on the rapid visual information processing task from the demand battery (*p* = .052 and *p* =.053 respectively). Whilst insignificant, those in the polysaccharide group exhibited a higher mean performance than those in the placebo condition (*p* = .08) but not the sucrose group (*p* = .14). Therefore, in analysis of post treatment change from baseline scores on the RVIP task, baseline performance was used as a covariate.

**Table 2: Means and Standard Deviations for baseline mood and well-being measures.**

| | **Treatment Condition** | | |
|---|---|---|---|
| | Polysaccharides N= 23 | Sucrose N= 24 | Placebo N= 26 |
| ***Profile of Mood States (POMS)*** | | | |
| Anxiety** | 5.73(5.26) | 5.76(6.04) | 2.39(3.99) |
| Tension | 7.26 (5.79) | 6.92 (4.77) | 6.56(3.23) |
| Depression | 6.34 (8.1) | 5.76 (5.88) | 4.04 (5.55) |
| Vigor | 17.17 (6.76) | 17.38 (6.13) | 18.17 (6.02) |
| Confusion | 6.21 (4.90) | 5.46 (4.31) | 4.82 (3.68) |
| Fatigue | 6.65 (5.14) | 6.42 (5.54) | 6.08 (4.88) |
| Total Mood Disturbance | 15.04 (29.31) | 12.96 (26.56) | 5.08 (19.28) |

| ***SF-36*** | | | |
|---|---|---|---|
| Physical Functioning | 27.72 (2.71) | 27.61 (2.15) | 27.17(3.29) |
| Role - physical | 7.36(1.09) | 7.03(1.48) | 6.95 (1.52) |
| Social Functioning | 8.95(1.73) | 9.03(1.34) | 8.86(1.68) |
| Role-Emotional | 5.22(1.02) | 5.34 (1.12) | 5.73 (.54) |
| General health | 18.31 (2.67) | 18.15 (2.16) | 17.95(2.49) |
| Vitality | 16.27 (4.48) | 16.80 (3.29) | 17.00 (3.72) |
| Health transition | 3.04 (.48) | 2.76 (.65) | 2.60 (.89) |
| Mental health | 24.27 (3.83) | 25.20 (3.22) | 24.30 (4.47) |
| Bodily pain | 8.82 (2.84) | 10.32 (1.83) | 9.04 (3.00) |

| | | | |
|---|---|---|---|
| ** F (2, 71) = 3.25, *p* = .045. | | | |

Effects of acute supplementation: Memory: The post-dose 'change-from-baseline' scores are presented in Table 3. There was a significant difference between groups in change from baseline performance on tasks of recognition memory (*F* (3,68) = 5.89, *p* = .004), list A (*F* (3,68) = 3.17, *p* = .044) and List B (*F* (3,68) = 5.17, *p* =.009) respectively. Post hoc analysis showed that those in the polysaccharide group recognized significantly more words overall than those in the sucrose group (*p* = .004) only, post treatment. Specifically, those in the polysaccharide condition recognized significantly more words from List B than those in the sucrose group (*p* = 0. 007). There was a trend for those in the polysaccharide condition to recognise more words from list A compared to sucrose (*p*= .083), but not placebo.

Cognitive demand: Serial 3s: There was a significant main effect of time on the number of correct answers (*F* (5, 340) = 2.16, *p* = .05), and trend towards a main effect of treatment (*F* (2, 68) = 2.70, *p* = .07), with the direction of means indicating a higher number correct responses in the polysaccharide group compared to those in the sucrose group (*p* = .07) but not placebo. There was no significant time by treatment interaction (*F* (10, 340) = .94, *p* = .48).

There was a significant main effect of time on the proportion of correct answers given average reaction time (*F* (5, 340) = 3.94, *p* = .002), and a trend towards a main effect of treatment (*F* (2, 70) = 2.96, *p* = .058), with the direction of means indicating a higher proportion of correct responses given average reaction time in the polysaccharide group compared to those in the sucrose group (*p* = .058) but not placebo. There was no significant time by treatment interaction (*F* (10, 340) = 0.898, *p* = 0.54).

There were no statistically significant effects associated with either time, treatment, or time by treatment interactions on the number of incorrect responses made or average reaction.

Serial 7s: There was a significant main effect of time on the number of correct answers made (*F*(5, 340) = 2.52, *p* = .02), but no significant main effect of treatment (*F* (2, 68) = .547, *p* = .58). There was a significant time by treatment interaction (*F* (10, 340) = 2.53, *p* = .006). Pairwise comparisons showed a significant difference between groups at 20 min with those in polysaccharide group making significantly more correct responses than the placebo group (*p* = .02) and those in the sucrose group making significantly more correct responses than the placebo group (*p* = .04).

There was no significant main effect of time on the proportion of correct answers given average reaction time (*F* (5, 340) = 1.66, *p* = 0.14), nor a main effect of treatment (*F* (2,68) =0.93, *p* = 0.39). There was a significant time by treatment interaction (*F* (10, 340) = 1.91, *p* = 0.04). Pairwise comparisons showed a significant difference between groups at 20 min with those in polysaccharide group making significantly more correct responses than the placebo group (p = .03), but not sucrose.

There were no statistically significant effects associated with either time, treatment, or time by treatment interactions on the number of incorrect responses made or average reaction.

RVIP: There were no statistically significant effects associated with either time, treatment or time by treatment interactions on the total number of responses, number of correct or incorrect responses made.

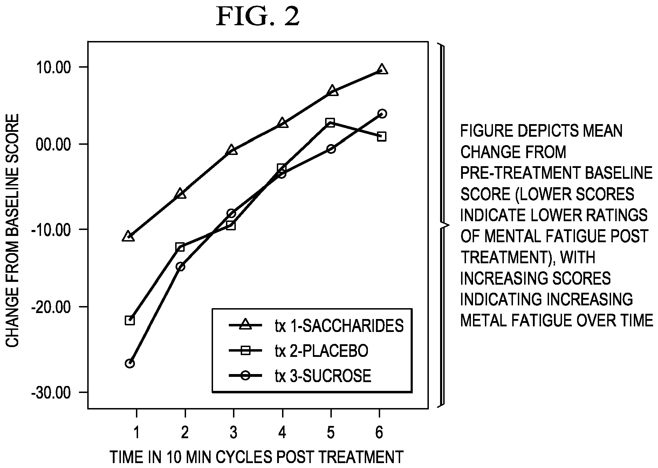

Mental Fatigue: There was a main effect of time on subjective ratings of mental fatigue (*F* (5, 340) = 75.44, *p* <.001), with increasing fatigue across time, shown in FIG. 2. There was no significant main effect of treatment (*F* (2, 68) = 2.04, *p* = .13) or time by treatment interaction (*F* (10, 340) = 1.39, *p* = .18). Pairwise comparisons showed a significant difference between groups at 10 min (*F* (2, 70) = 4.27, *p* = .018), with those in the polysaccharide group reporting a significant reduction in feelings of fatigue approximately 40 minutes post consumption, compared to those in the sucrose group (*p* = .01)

Mood: There were no statistically significant effects associated with either treatment on subjective levels of anxiety measured by the State-trait questionnaire, or on the Bond-lader scales of alertness, contentedness, and calmness as change from baseline scores.

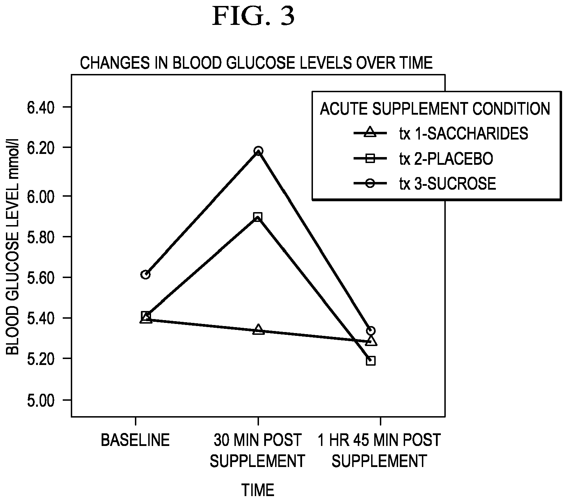

Blood glucose measurement: There was a significant main effect of time (*F* (2, 140) = 37.28, *p* <.001) and significant interaction of time and treatment condition (*F* (4,140) = 8.39, *p* = <.001). There was no significant main effect of treatment condition (*F*(2, 70) = 2.41, *p* = .09). Pairwise comparisons show that at 30 minutes post ingestion of supplement, those who consumed the polysaccharide supplement had significant lower blood glucose response than those who had consumed placebo (*p* = .038) and sucrose (*p*<.001). Specifically, there was no rise in blood glucose response following polysaccharide intake compared to the blood glucose response or those individuals who consumed sucrose and placebo, as shown in FIG. 3.

**Table 3: Means and Standard deviations for RAVLT post-dose change from baseline scores.**

| **Measure** | **Treatment Condition** | | | **F(3,68)** |
|---|---|---|---|---|
| | Polysaccharides N= 23 | Sucrose N= 24 | Placebo N= 26 | |
| ***RAVLT*** | | | | |
| Trial 1 | -0.78 (1.78) | -0.26 (2.23) | -0.54(1.81) | 0.429 |
| Trial 2 | -0.60 (2.08) | -0.57 (2.13) | -0.95 (2.47) | 0.017 |
| Trial 3 | 0.21 (1.95) | -0.42 (1.83) | -0.58 (1.55) | 1.35 |
| Trial4 | -0.04 (2.05) | -0.42 (1.72) | -0.08 (2.20) | 0.263 |
| Trial 5 | 0.08 (1.59) | -0.19 (1.72) | 0.58 (1.74) | 0.918 |
| Sum Immediate Recall | -1.13(5.54) | -1.88 (5.83) | -1.58 (6.00) | 0.103 |
| Trial 6 (List B) | -0.08 (2.02) | -0.84 (2.16) | -0.25 (2.11) | 0.821 |
| Trial 7 | -0.82 (2.90) | -1.53 (2.37) | -1.08 (3.29) | 0.369 |
| Trial 8 | -1.91 (3.02) | -3.23 (2.80) | -2.29 (3.23) | 1.18 |
| Sum delayed recall | -2.73 (5.21) | -4.76 (4.18) | -3.37(5.56) | |
| Recog A | -0.26 (3.29) | -2.38 (2.46) | -2.37 (4.11) | 3.28* |
| Recog B | -0.26 (3.10) | -3.38 (3.55) | -1.45 (3.61) | 4.99* |
| Sum Recognition | -0.52 (5.24) | -5.76 (4.91) | -3.83 (6.07) | 5.89* |
| Interference (trial 7 - trial 5) | -0.91 (2.77) | -1.34 (2.18) | -1.66 (2.88) | 0.417 |
| Learning (trial 5- trial 1) | 0.86 (2.20) | 0.07 (2.78) | 1.12 (2.43) | 1.20 |
| Forgetting (trial 8 - trial 7) | -1.08 (2.82) | -1.69 (3.06) | -1.20 (3.42) | 0.254 |

| | | | | |
|---|---|---|---|---|
| *p= <.05 | | | | |

The present study describes the acute effects of plant-saccharides (Ambrotose® Complex) on cognition and mood in middle-aged adults. The study presented herein is used to determine whether saccharides have positive acute effects on memory, cognition, well-being, and mood and whether any effects may be explained through glycaemic effects, for example the provision of glucose as a metabolic substrate. The methodology used provided a novel design to examine the effects of treatment (saccharides, sucrose, and placebo) between subjects across highly effortful and mentally fatiguing conditions.

The results presented hereinabove show a significant effect of plant polysaccharide consumption on recognition memory performance compared to sucrose consumption. Specifically, despite increasing subjective ratings of mental fatigue, recognition of words previously learned was significantly higher for those who consumed plant-polysaccharides compared to sucrose. This positive effect on recognition memory performance suggests that those who received plant-polysaccharides were aided in encoding the information presented and could retrieve that information, when presented with the words as a cue, more readily.

With regard to performance on tasks in the cognitive demand battery, there was a trend towards a treatment effect of polysaccharides on the number and proportion of Serial Three subtractions made (*p* = .07 and .058 respectively). However, the only significant time and treatment interaction was obtained for Serial Seven subtractions. Specifically, there was a significant interaction between treatment conditions and the number of correct responses made. Importantly, pairwise comparisons showed a significantly greater number of correct responses being performed at the 2nd cycle of the battery (approximately 1 hour post consumption) for those in the polysaccharide condition compared to those in the placebo condition.

Performance on Serial Sevens subtraction tasks is rated as significantly more demanding than Serial Threes (Kennedy and Scholey, 2000), and relies more heavily on working memory and executive resources. The current results suggest that consumption of plant polysaccharides maintains working memory performance despite conditions of mental fatigue. Interestingly, the trends towards improved performance on Serial Threes following intake of plant-polysaccharides, may indicate potential working memory and attention effects, as performance on both serial three and serial seven tasks have attention components.

In relation to mood, there was no significant interaction between treatment condition and changes in subjective ratings of feelings of anxiety, alertness, contentedness or calmness.

Importantly, the recognition memory and working memory effects were observed following plant-polysaccharide intake, independent of blood glucose response. The absence of a raised blood glucose following intake of plant-polysaccharides indicates that the memory effects were not directly related to the modulation of blood glucose levels.

In conclusion, this is a first of kind study of the acute benefits for cognitive function associated with plant-polysaccharide consumption in healthy middle-aged adults, independent of blood glucose response. These findings suggest that the consumption of 4 g of plant polysaccharides may be beneficial for recognition memory performance and cognitive tasks reflecting working memory and executive function during highly demanding and mentally fatiguing conditions. The results of the present invention may have implications for individuals who require a memory 'boost' to cope with the demands that a busy, taxing lifestyle places upon cognitive resources.

It is contemplated that any embodiment discussed in this specification can be implemented with respect to any method, kit, reagent, or composition, and vice versa. Furthermore, compositions of the invention can be used to achieve methods of the disclosure.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

## Claims

1. A dietary supplement for use in a method of improving recognition memory performance in a human subject by
selecting a human subject in need of improvement of recognition memory performance; and
administering to the human subject a nutritionally effective amount of the dietary supplement in an amount sufficient to improve recognition memory performance;
waiting 30 minutes to allow the dietary supplement to be absorbed; and
obtaining an improvement in recognition memory performance in the human subject compared to a recognition memory performance in a human subject who consumed sucrose or a placebo as a dietary supplement wherein the improvement in recognition memory performance occurs within 30 minutes to two hours after the dietary supplement has been administered;
wherein the dietary supplement comprises a nutritionally effective amount of isolated and purified acetylated mannose; and
a nutritionally effective amount of at least five isolated and purified saccharides selected from the group consisting of: galactose, glucose, mannose, xylose, N-acetylneuraminic acid, fucose, N-acetylgalactosamine, N-acetylglucosamine, arabinose, glucuronic acid, galacturonic acid, iduronic acid and arabinogalactan.

2. The dietary supplement for use in the method according to claim 1, wherein the dietary supplement is orally administered.

3. The dietary supplement for use in the method according to claim 1 or 2, wherein the dietary supplement is a powder, is dissolved in a liquid, is encapsulated, or any combinations thereof.

4. The dietary supplement for use in the method according to claims 1 to 3, further comprising the step of measuring blood glucose levels prior to and after administration of the dietary supplement.

5. The dietary supplement for use in the method according to claims 1 to 4, wherein the dietary supplement is administered simultaneously or sequentially in one or a combination of dosage forms.

6. The dietary supplement for use in the method according to claim 1 to 5, wherein the dietary supplement is administered simultaneously or sequentially with one or more drugs, vitamins, other nutritional supplements, or any combinations thereof.

7. The dietary supplement for use in the method according to claims 1 to 6, wherein the dietary supplement does not cause an elevated blood glucose level in the human subject.

8. The dietary supplement for use in the method according to claims 1 to 6, further comprising the step of terminating the administration of the dietary supplement in case of an elevated blood glucose level.

## Patentansprüche

1. Nahrungsergänzungsmittel zur Anwendung in einem Verfahren zum Verbessern der Leistung des Wiedererkennungsgedächtnisses bei einem menschlichen Individuum durch Folgendes
Auswählen eines menschlichen Individuums, welches die Verbesserung des Wiedererkennungsgedächtnisses benötigt, und
Verabreichen einer ernährungsphysiologisch wirksamen Menge des Nahrungsergänzungsmittels an das menschliche Individuum in einer zum Verbessern der Leistung des Wiedererkennungsgedächtnisses ausreichenden Menge;
Abwarten für 30 Minuten, um die Aufnahme des Nahrungsergänzungsmittel zu ermöglichen; und
Erhalten einer Verbesserung der Leistung des Wiedererkennungsgedächtnisses bei dem menschlichen Individuum im Vergleich zu einem menschlichen Individuum, das Saccharose oder ein Placebo als Nahrungsergänzungsmittel eingenommen hat, wobei die Verbesserung der Leistung des Wiedererkennungsgedächtnisses innerhalb von 30 Minuten bis zwei Stunden nach dem Verabreichen des Nahrungsergänzungsmittels auftritt;
wobei das Nahrungsergänzungsmittel eine ernährungsphysiologisch wirksame Menge von isolierter und gereinigter Mannose und
eine ernährungsphysiologisch wirksame Menge von mindestens fünf isolierten und gereinigten Sacchariden, ausgewählt aus der Gruppe, bestehend aus: Galactose, Glucose, Mannose, Xylose, N-Acetylneuraminsäure, Fucose, N-Acetylgalactosamin, N-Acetylglucosamin, Arabinose, Glucuronsäure, Galacturonsäure, Iduronsäure und Arabinogalactan, aufweist.

2. Nahrungsergänzungsmittel zur Anwendung in dem Verfahren nach Anspruch 1, wobei das Nahrungsergänzungsmittel oral verabreicht wird.

3. Nahrungsergänzungsmittel zur Anwendung in dem Verfahren nach Anspruch 1 oder 2, wobei das Nahrungsergänzungsmittel ein Pulver ist, in einer Flüssigkeit gelöst ist, eingekapselt ist oder eine Kombinationen davon ist.

4. Nahrungsergänzungsmittel zur Anwendung in dem Verfahren nach Anspruch 1 bis 3, das ferner den Schritt des Messens des Blutzuckerspiegels vor und nach der Verabreichung des Nahrungsergänzungsmittels aufweist.

5. Nahrungsergänzungsmittel zur Anwendung in dem Verfahren nach Anspruch 1 bis 4, wobei das Nahrungsergänzungsmittel gleichzeitig oder der Reihe nach in einer oder einer Kombination von Darreichungsformen verabreicht wird.

6. Nahrungsergänzungsmittel zur Anwendung in dem Verfahren nach Anspruch 1 bis 5, wobei das Nahrungsergänzungsmittel gleichzeitig oder der Reihe nach mit einem oder mehreren Arzneimitteln, Vitaminen, anderen Nahrungsergänzungen oder einer Kombinationen davon verabreicht wird.

7. Nahrungsergänzungsmittel zur Anwendung in dem Verfahren nach Anspruch 1 bis 6, wobei das Nahrungsergänzungsmittel keinen erhöhten Blutzuckerspiegel bei einem menschlichen Individuum bewirkt.

8. Nahrungsergänzungsmittel zur Anwendung in dem Verfahren nach Anspruch 1 bis 6, das ferner den Schritt des Beendens der Verabreichung des Nahrungsergänzungsmittels im Falle eines erhöhten Blutzuckerspiegels aufweist.

## Revendications

1. Complément alimentaire pour son utilisation dans un procédé destiné à améliorer les performances de la mémoire de reconnaissance chez un sujet humain en
sélectionnant un sujet humain ayant besoin d'améliorer les performances de sa mémoire de reconnaissance ; et
administrant au sujet humain une quantité efficace d'un point de vue nutritionnel du complément alimentaire en une quantité suffisante pour améliorer les performances de la mémoire de reconnaissance ;
attendant 30 minutes pour permettre l'absorption du complément alimentaire ; et
obtenant une amélioration des performances de la mémoire de reconnaissance chez le sujet humain par rapport à des performances de la mémoire de reconnaissance chez un sujet humain qui a consommé du saccharose ou un placébo en tant que complément alimentaire, dans lequel l'amélioration des performances de la mémoire de reconnaissance se produit dans les 30 minutes à deux heures après l'administration du complément alimentaire ;
dans lequel le complément alimentaire comprend une quantité efficace d'un point de vue nutritionnel de mannose acétylé isolé et purifié ; et
une quantité efficace d'un point de vue nutritionnel d'au moins cinq saccharides isolés et purifiés sélectionnés parmi le groupe constitué du galactose, du glucose, du mannose, du xylose, de l'acide N-acétylneuraminique, du fucose, de la N-acétylgalactosamine, de la N-acétylglucosamine, de l'arabinose, de l'acide glucuronique, de l'acide galacturonique, de l'acide iduronique et de l'arabinogalactane.

2. Complément alimentaire pour son utilisation dans le procédé selon la revendication 1, dans lequel le complément alimentaire est administré par voie orale.

3. Complément alimentaire pour son utilisation dans le procédé selon la revendication 1 ou 2, dans lequel le complément alimentaire est une poudre, est dissous dans un liquide, est encapsulé, ou toute combinaison de ceux-ci.

4. Complément alimentaire pour son utilisation dans le procédé selon les revendications 1 à 3, comprenant en outre l'étape consistant à mesurer la glycémie avant et après l'administration du complément alimentaire.

5. Complément alimentaire pour son utilisation dans le procédé selon les revendications 1 à 4, dans lequel le complément alimentaire est administré simultanément ou séquentiellement en une ou une combinaison de formes posologiques.

6. Complément alimentaire pour son utilisation dans le procédé selon les revendications 1 à 5, dans lequel le complément alimentaire est administré simultanément ou séquentiellement avec un ou plusieurs médicaments, vitamines, ou autres compléments alimentaires, ou toute combinaison de ceux-ci.

7. Complément alimentaire pour son utilisation dans le procédé selon les revendications 1 à 6, dans lequel le complément alimentaire ne provoque pas d'augmentation de la glycémie chez le sujet humain.

8. Complément alimentaire pour son utilisation dans le procédé selon les revendications 1 à 6, comprenant en outre l'étape consistant à mettre fin à l'administration du complément alimentaire en cas d'augmentation de la glycémie.
